Europäisches Patentamt

European Patent Office

Office européen des brevets

(11) Veröffentlichungsnummer: **0 301 322**
**A1**

(12) **EUROPÄISCHE PATENTANMELDUNG**

(21) Anmeldenummer: 88111262.7

(51) Int. Cl.⁴: **A61B 5/04**

(22) Anmeldetag: 13.07.88

(30) Priorität: 27.07.87 DE 3724842

(43) Veröffentlichungstag der Anmeldung:
01.02.89 Patentblatt 89/05

(84) Benannte Vertragsstaaten:
DE FR IT NL

(71) Anmelder: Siemens Aktiengesellschaft Berlin
und München
Wittelsbacherplatz 2
D-8000 München 2(DE)

(72) Erfinder: Schneider, Siegfried, Dr.
Kulmbacher Strasse 33
D-8520 Erlangen(DE)

(54) **Einrichtung zur Stimulation von optischen Reizen.**

(57) Die Einrichtung dient dazu, in einem Patienten optisch evozierte Reize auszulösen, die mittels einer Meßeinrichtung (10) erfaßt werden. Die Einrichtung umfaßt einen Stimulationssignalgeber (12) mit einer Fläche (14), auf der optische Stimulationssignale dargestellt werden. Vor der Fläche (14) ist ein erstes Linsensystem (16) angeordnet, das das Bild auf das eine Ende (18) eines Lichtwellenleiters (20) abbildet. Am anderen Ende (24) des Lichtwellenleiters (20) ist ein zweites Linsensystem (26) angeordnet. Dieses bildet die übertragenen Stimulationssignale auf einer Darstellungsfläche (28) ab. Das zweite Linsensystem (26) ist an einer Halterung (30) befestigt, die am Kopf (2) des Patienten derart angebracht werden kann, daß das Auge des Patienten auf die Darstellungsfläche (28) blickt und damit den optischen Stimulationssignalen ausgesetzt ist. Als Materialien für die Halterung (30), das zweite Linsensystem (26) und den Lichtleiter (20) werden nichtmagnetische Materialien verwendet. Die Einrichtung gewährleistet eine störsichere Messung biomagnetischer Signale bei optisch evozierten Reizen.

## Einrichtung zur Stimulation von optischen Reizen

Die Erfindung bezieht sich auf eine Einrichtung zur Stimulation von optischen Reizen in einem Patienten mit einem optischen Stimulationssignalgeber, dessen Stimulationssignale dem Auge des Patienten zuführbar sind.

Die Messung biomagnetischer Signale gewinnt für die medizinische Diagnostik immer größere Bedeutung (vgl. Zeitschrift "Bild der Wissenschaft", Heft 8, 1986, Seiten 76 - 83). Mit Hilfe von SQUID (Super Conducting Quantum Interference Device)-Systemen lassen sich die äußerst schwachen biomagnetischen Signale meßtechnisch erfassen, z.B. die der sog. evozierten Magnetfelder des menschlichen Gehirns, die in der Größenordnung von nur $10^{-14}$ T liegen. Der Proband ist dabei in einem magnetisch abgeschirmten Meßraum untergebracht, und sein Kopf wird mit dem SQUID-System berührungslos abgetastet. Die mit Hilfe der SQUID-Technologie gewonnenen Daten werden sodann mit Hilfe eines Computers unter Zugrundelegung gewisser mathematischer Modelle ausgewertet.

Von besonderem Interesse für die experimentelle Forschung und die Diagnostik sind durch Hör- oder Sehreize induzierte magnetische Felder im Gehirn. Eine hierbei verwendete Einrichtung zur Stimulation optischer Reize muß gewissen Anforderungen genügen.

Zunächst einmal ist zu berücksichtigen, daß die Messung von optisch evozierten Potentialen oder Magnetfeldern am Patienten in besonderer Umgebung durchgeführt wird. Hierbei kann es sich insbesondere um eine elektrische oder magnetische Abschirmkammer, um das Magnetfeld eines MR-Magneten oder um die Nähe von Diagnose-und/oder Therapiegeräten (u.a. um Monitoring-, Chirurgie-, mit Gas arbeitende Geräte) handeln.

Dann ist zu berücksichtigen, daß die Messung mit möglichst wenig Störeinflüssen durchgeführt werden soll. Es sollen also möglichst wenig Störungen auf die Meßapparatur der elektrischen Potentiale oder Magnetfelder (z.B. ein SQUID-System), auf die Einrichtung zur Stimulation selbst, auf das Diagnosegerät (z.B. eine MR-Aufnahme-Einheit) und das Therapiegerät und schließlich auch auf den Probanden einwirken. Gerade das Entspanntsein und die Konzentration des Probanden sind notwendig für eine gute Signalqualität.

Schließlich soll die Messung nach Möglichkeit auch mit einer möglichst großen Vielfalt oder Variationsbreite beim Stimulieren durchzuführen sein, um auch höhere Hirnfunktionen des Probanden testen zu können.

Die optische Evozierung wurde bisher hauptsächlich nach zwei Prinzipien durchgeführt:

a) Es wurde dem Probanden ein Schachbrettmuster gezeigt, d.h. ein Feld von hellen und dunklen Feldern, das mit einem diaprojektor-ähnlichen Gerät, mit einem speziell angesteuerten Fernsehmonitor oder mittels einer LED-Matrix erzeugt wurde. Dieses Schachbrettmuster wurde vom Probanden direkt betrachtet. Durch die Nähe der das Schachbrettmuster erzeugenden Einrichtung waren Störeinflüsse nicht ausgeschlossen.

b) Das Auge des Probanden wurde Lichtblitzen ausgesetzt, die mit einer Blitzlampe erzeugt wurden. Hierbei handelte es sich um einen groben Funktionstest der optischen Signalverarbeitung im Gehirn. Eine Mitarbeit des Patienten, insbesondere durch Fokussierung auf einen bestimmten Punkt, war nicht notwendig. Deshalb konnte bei diesem Prinzip auch eine Brille benutzt werden, bei der die Lichtblitze direkt vor dem Auge erzeugt werden.

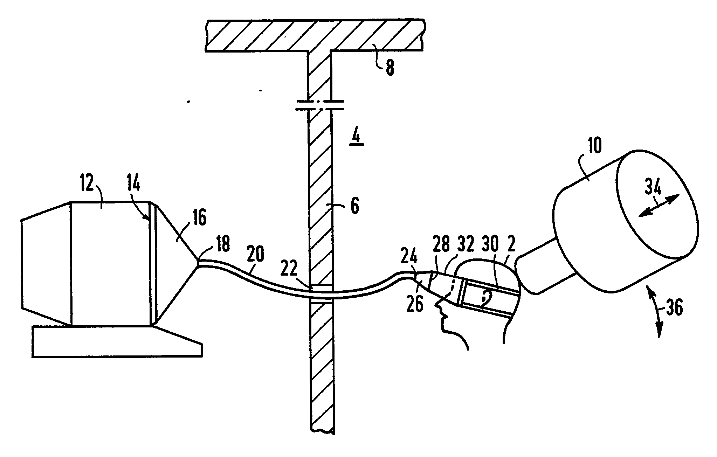

Auf einem anderen Arbeitsgebiet, nämlich dem der Flugsimulation zur Schulung von Piloten, ist es an sich bekannt, eine Projektionsfläche in den Pilotenhelm einzubauen; das dem Piloten dar gebotene Bild wird von einem Projektor hinter dem Piloten erzeugt und durch Glasfaserstränge übertragen (vgl. "Spektrum der Wissenschaft", Sept. 1986, Seiten 56 - 64, insbes. Bild 3 auf S. 59).

Der Erfindung liegt die Aufgabe zugrunde, eine Einrichtung der eingangs genannten Art so auszubilden, daß sie sich bei der Messung von optisch evozierten Potentialen oder Magnetfeldern am Patienten in besonderer Umgebung (z.B. Abschirmkammer, im Magnetfeld eines MR-Magneten oder in der Nähe von Diagnose-und/oder Therapiegeräten) einsetzen läßt und dabei zu möglichst wenig Störungen bei der Messung führt.

Diese Aufgabe wird erfindungsgemäß dadurch gelöst, daß der Stimulationssignalgeber eine Fläche aufweist, auf der die Stimulationssignale optisch darstellbar sind, daß vor der Fläche ein erstes Linsensystem angeordnet ist, das die Stimulationssignale auf das eine Ende eines Lichtwellenleiters abbildet, daß am anderen Ende des Lichtwellenleiters ein zweites Linsensystem angeordnet ist, das die übertragenen Stimulationssignale auf einer Darstellungsfläche abbildet, daß das zweite Linsensystem an einer Halterung befestigt ist, die am Kopf des Patienten derart anbringbar ist, daß das Auge des Patienten auf die Darstellungsfläche blickt, und daß als Materialien zumindest für die Halterung, das zweite Linsensystem und den Lichtleiter nichtmagnetische Materialien eingesetzt sind.

Nach einer bevorzugten Ausführungsform ist als Stimulationssignalgeber eine Farbgrafikstation vorgesehen. Hierbei handelt es sich um einen schnellen Computer, der mit Hilfe eines vorgegebenen Programms eine farbige Grafik auf einem Bildschirm in Form eines stehenden oder bewegten Bildes erzeugt. Je nach Programm kann es sich bei dem erzeugten Stimulationssignal um eine gleichmäßige oder ungleichmäßige Struktur, um ein variables Bild o.dgl. handeln.

Weitere vorteilhafte Ausgestaltungen der Erfindung sind in den Unteransprüche gekennzeichnet.

Als Vorteile sind zu verzeichnen: Bei dieser Einrichtung werden die Stimulationssignale in größerer, durch die Länge des Lichtwellenleiters bestimmter Entfernung vom Patienten und von den Meß- und/oder Therapiegeräten erzeugt. Der Patient und die genannten Geräte werden dadurch relativ wenig gestört. Falls der Patient in einer Abschirmkammer untergebracht ist, ist in deren Wand nur eine kleine Durchtrittsöffnung für die Übertragung der optischen Signale mittels des Lichtwellenleiters erforderlich.

Wird als Stimulationssignalgeber die genannte Farbgrafikstation verwendet, dann können sehr viele verschiedene und auch sehr komplexe Reize im Patienten bei hoher Verarbeitungsgeschwindigkeit erzeugt werden.

Ausführungsbeispiele der Erfindung werden im folgenden anhand einer Figur näher erläutert.

Nach der Figur ist ein Patient, dessen Kopf mit 2 bezeichnet ist, in einer Abschirmkammer 4 untergebracht, von der lediglich eine Seitenwand 6 und die Decke 8 angedeutet sind. In dieser Abschirmkammer 4 befindet sich eine (perspektivisch gezeigte) Meßeinrichtung 10 zur Messung von optisch evozierten Potentialen oder Magnetfeldern. Hierbei kann es sich insbesondere um ein SQUID-Magnetometer bekannter Bauart handeln.

Die dargestellte Einrichtung zur Stimulation von optischen Reizen umfaßt einen Stimulationssignalgeber 12, der eine Bildebene oder Fläche 14 aufweist, auf der optische Stimulationssignale flächenhaft darstellbar sind. Die Fläche 14 kann somit auch als Bildebene für die vom Stimulationssignalgeber 12 erzeugten Bilder in Form von Strukturen, Symbolen, etc. bezeichnet werden. Bevorzugt ist der Stimulationssignalgeber 12 eine sog. Farbgrafikstation, also ein schneller Grafik-Computer in Verbindung mit einem Bildschirm, auf dem - je nach Programmierung - ein stehendes oder bewegtes Bild erzeugt werden kann.

Vor der Fläche 14 ist ein erstes Linsensystem 16 angeordnet, das das erzeugte Bild auf das eine Ende 18 eines längeren Lichtwellenleiters 20 abbildet. Dieser Lichtwellenleiter 20 ist flexibel und durch eine Öffnung 22 in der Wand 6 der Abschirmkammer 4 geführt. Am anderen Ende 24 des Lichtwellenleiters 20 ist ein zweites Linsensystem 26 angeordnet. Dieses zweite Linsensystem 26 bildet die übertragenen Stimulationssignale auf einer Darstellungfläche 28 ab, und zwar so, daß das hier erzeugte zweite Bild kleiner ist als das auf der Fläche 14 erzeugte erste Bild. Wie ersichtlich, ist das erste Linsensystem 16 zu einer verkleinerten Abbildung und das zweite Linsensystem 26 zu einer vergrößerten Abbildung vorgesehen.

Das zweite Linsensystem 26 ist am Kopf 2 des Patienten derart angebracht, daß das Auge des Patienten auf die Darstellungsfläche 28 blickt. Hierzu dient eine Halterung 30 aus einem Nicht-Metall. Zur Wahrung eines gewissen Abstands zwischen dem Auge des Patienten und der Darstellungsfläche 28 ist zwischen letzterer und der Halterung 30 ein Lichtschacht oder Lichtkanal 32 angeordnet, der auch vor seitlichem Lichteinfall schützt. Auch dieser Lichtschacht 32 ist bevorzugt aus einem Nicht-Metall gefertigt. Als Materialien zumindest für die Halterung 30, den Lichtschacht 32, das zweite Linsensystem 26 und den Lichtwellenleiter 20 sind nichtmagnetische Materialien eingesetzt, um Störungen bei der Messung der biomagnetischen Signale zu vermeiden.

Als Halterung 30 könnte prinzipiell eine Helm verwendet werden, wie er bei Motorradfahrern oder Piloten bekannt ist. Statt dessen könnte auch ein brillenartiges Gestell verwendet werden. Dies ist in der Figur angedeutet. Das Gestell weist dabei ein Band auf, das z.B. aus Gummi oder Plastik besteht und das um den Kopf 2 des Patienten schlingbar ist. Insgesamt ergibt sich ein fester Halt am Kopf 2, so daß sich der Patient voll auf die übertragenen optischen Signale konzentrieren kann, die er auf der Darstellungsfläche 28 sieht.

Bevorzugt ist die Meßeinrichtung 10, also beispielsweise das erwähnte SQUID-Magnetometer, relativ zur Halterung 30 mittels einer Bewegungseinrichtung bewegbar. Dies ist durch einen geraden und einen gekrümmten Doppelpfeil 34 bzw. 36 schematisch angedeutet. Auf diese Weise lassen sich berührungsfrei die optisch evozierten Potentiale bzw. Magnetfelder im Inneren des Kopfes 2 messen.

Dadurch, daß der Stimulationssignalgeber 12 über die Linsensysteme 16, 26 und den Lichtwellenleiter 22 relativ weit vom Patienten und von der Meßeinrichtung 10 sowie ggf. weiterer Diagnose- und/oder Therapiegeräte und auch außerhalb der Abschirmkammer 4 angeordnet ist, können die optischen Reizsignale im Hirn des Patienten störungsfrei gemessen werden.

## Ansprüche

1. Einrichtung zur Stimulation von optischen Reizen in einem Patienten mit einem optischen Stimulationssignalgeber, dessen Stimulationssignale dem Auge des Patienten zuführbar sind, **dadurch gekennzeichnet,**daß der Stimulationssignalgeber (12) eine Fläche (14) aufweist, auf der die Stimulationssignale optisch darstellbar sind, daß vor der Fläche (14) ein erstes Linsensystem (16) angeordnet ist, das die Stimulationssignale auf das eine Ende (18) eines Lichtwellenleiters (20) abbildet, daß am anderen Ende (24) des Lichtwellenleiters (20) ein zweites Linsensystem (26) angeordnet ist, das die übertragenen Stimulationssignale auf einer Darstellungsfläche (28) abbildet, daß das zweite Linsensystem (26) an einer Halterung (30) befestigt ist, die am Kopf (2) des Patienten derart anbringbar ist, daß das Auge des Patienten auf die Darstellungsfläche (28) blickt, und daß als Materialien zumindest für die Halterung (30), das zweite Linsensystem (26) und den Lichtleiter (20) nichtmagnetische Materialien eingesetzt sind.

2. Einrichtung nach Anspruch 1, **dadurch gekennzeichnet,** daß als Stimulationssignalgeber (12) eine Farbgrafikstation vorgesehen ist.

3. Einrichtung nach Anspruch 1 oder 2, **dadurch gekennzeichnet,** daß die Halterung (30) ein Helm ist.

4. Einrichtung nach Anspruch 1 oder 2, **dadurch gekennzeichnet,** daß die Halterung (30) ein brillenartiges Gestell ist.

5. Einrichtung nach Anspruch 4, **dadurch gekennzeichnet,** daß das Gestell ein Band aufweist, das um den Kopf (2) des Patienten schlingbar ist.

6. Einrichtung nach einem der Ansprüche 1 bis 5, **dadurch gekennzeichnet,** daß zwischen der Darstellungsfläche (28) und der Halterung (30) ein Lichtschacht (32) angeordnet ist, der vor seitlichem Lichteinfall schützt.

7. Einrichtung nach einem der Ansprüche 1 bis 6, **dadurch gekennzeichnet,** daß das erste Linsensystem (16) zu einer verkleinerten Abbildung und das zweite Linsensystem (26) zu einer vergrößerten Abbildung vorgesehen ist.

8. Einrichtung nach einem der Ansprüche 1 bis 7, **da durch gekennzeichnet,** daß zur Messung der im Gehirn des Patienten optisch ausgelösten Reize eine Meßeinrichtung (10), insbesondere ein SQUID-Magnetometer, vorgesehen ist.

9. Einrichtung nach Anspruch 8, **dadurch gekennzeichnet,** daß die Meßeinrichtung (10) relativ zur Halterung (30) bewegbar ist.

10. Einrichtung nach einem der Ansprüche 1 bis 9, **dadurch gekennzeichnet,** daß der Lichtwellenleiter (20) durch eine Wand (6) geführt ist.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | G.J. STOK: "The inverse problem in EEG and MEG with application to visual evoked responces", 1986, Seiten 41-47, Krips, Repro Meppel, Hengelo, NL * Seite 45, Figuren 3,4 * --- | 1 | A 61 B 5/04 |
| A | US-A-4 457 716 (EBERHAUT et al.) * Zusammenfassung * --- | 1 | |
| A | US-A-4 340 274 (A.M. SPOONER) * Zusammenfassung * ----- | 1 | |

**RECHERCHIERTE SACHGEBIETE (Int. Cl.4)**

B 61 B
G 09 B

Der vorliegende Recherchenbericht wurde für alle Patentansprüche erstellt

| Recherchenort | Abschlußdatum der Recherche | Prüfer |
|---|---|---|
| DEN HAAG | 04-11-1988 | ARGENTINI A. |